# EUROPEAN PATENT SPECIFICATION

(11) **EP 4 239 333 B1**
(45) Date of publication and mention of the grant of the patent: **15.07.2026**
(21) Application number: 22160066.1
(22) Date of filing: 03.03.2022
(51) Int. Cl.: G01N 33/50, G01N 33/569, G01N 33/68

(54) **METHOD FOR SELECTING CLONAL CELLS EXPRESSING CORRECTLY ASSEMBLED MULTI-SPECIFIC BINDING MOLECULES**
VERFAHREN ZUR SELEKTION KLONALER ZELLEN, DIE KORREKT ZUSAMMENGEFÜGTE MULTISPEZIFISCHE BINDUNGSMOLEKÜLE EXPRIMIEREN
PROCÉDÉ DE SÉLECTION DE CELLULES CLONALES EXPRIMANT DES MOLÉCULES DE LIAISON MULTISPÉCIFIQUES ASSEMBLÉES CORRECTEMENT

(43) Date of publication of application: 06.09.2023
(73) Proprietor: Lonza Biologics Plc., Slough, Berkshire SL1 4DX (GB)
(72) Inventor: GOMEZ DE LA CUESTA, Ramón, Cambridge, CB10 1XL (GB); FIC, Weronika, Cambridge, CB10 1XL (GB); SHARMA, Amit, Slough, Berkshire, SL1 4DX (GB)
(74) Representative: Greiner, Elisabeth

(56) References cited:
- WO-A1-2022/213077
- WO-A2-2018/076024
- CN-A- 104 818 295
- US-A1- 2005 266 425
- Translation of CN104818295A
- XU LIMING ET AL: "Bi-specific antibodies with high antigen-binding affinity identified by flow cytometry", INTERNATIONAL IMMUNOPHARMACOLOGY, ELSEVIER, AMSTERDAM, NL, vol. 24, no. 2, 16 December 2014 (2014-12-16), pages 463 - 473, XP029196224, ISSN: 1567-5769, DOI: 10.1016/J.INTIMP.2014.12.011
- WINTERS AARON ET AL: "Rapid single B cell antibody discovery using nanopens and structured light", MABS, vol. 11, no. 6, 11 June 2019 (2019-06-11), US, pages 1025 - 1035, XP055920304, ISSN: 1942-0862, Retrieved from the Internet <URL:https://tandfonline.com/doi/pdf/10.1080/19420862.2019.1624126> DOI: 10.1080/19420862.2019.1624126

## Description

### Field of and Background to the Invention

The present invention relates to methods for selecting cells that express multi-specific binding molecules, such as antibodies, and in particular bispecific antibodies, with high levels of correctly assembled protein.

Bispecific antibodies have shown much promise as a therapeutic approach: bispecific antibodies are entering clinical studies in record numbers, with most developed for cancer. Such molecules, unlike naturally occurring IgG molecules, contain at least 2 and usually at least 4 different chains (e.g., two heavy and two light chains). Therefore, there are a number of different permutations for assembly of the complete product, whereas for naturally occurring IgG molecules there is only one.

A number of solutions have been proposed to solve these chain pairing problems. Knob into holes is commonly used to direct heavy chain-heavy chain pairing. Various techniques have been used to direct heavy-light chain pairing, such as the CrossMabs approach. CN104818295A discloses methods for preparing and screening cell lines expressing bispecific antibodies. Nonetheless there is a need to select clonal production cell lines that efficiently express bi-specifics where a high level of correct assembly is achieved and correctly paired molecules can be readily separated from incorrectly paired molecules during downstream processing. The present invention fulfils this need.

### Summary of the Invention

The present invention provides a method that enables individual cell clones to be analysed *in vitro* whilst growing and secreting recombinant protein. By using reagents that bind specifically to the correctly paired regions of the product, different producing clones can be assessed and compared to enable selection of cells that efficiently produce improved ratios of correctly formed product to incorrectly formed product.

Accordingly, in a first aspect, the present invention provides a method of selecting a cell for expression of a multi-specific binding molecule comprising the following steps:
(a) providing a population of host cells comprising one or more nucleic acid sequences encoding at least two polypeptides,
   wherein the polypeptides are expressed by the host cells; and
   wherein a first polypeptide forms a first binding site for a target molecule, and a second polypeptide forms a second binding site for a target molecule, which is different to the first binding site;
(b) contacting the host cells with (i) a first labelled reagent that binds selectively to the first binding site, and (ii) a second labelled reagent that binds selectively to the second binding site, wherein the labels for the first and second reagents are different;
(c) measuring the level of first labelled reagent and second labelled reagent bound to their respective binding sites; and
(d) selecting one or more host cells expressing a multi-specific binding molecule based on a comparison of the two levels measured in step (c); and
wherein the host cells are cultured in a microfluidic device, wherein the microfluidic device comprises a microfluidic channel to which a plurality of sequestration pens are fluidically connected, wherein the host cells are loaded into the microfluidic device such that a plurality of the sequestration pens are each loaded with one host cell.

In one embodiment, the first polypeptide and a third polypeptide together form the first binding site for a target molecule, and the second polypeptide and a fourth polypeptide together form the second binding site for a target molecule, which is different to the first binding site.

In one embodiment, the third and fourth polypeptides are the same.

In one embodiment, the multi-specific binding molecules are secreted by the host cells.

In one embodiment, the multi-specific binding molecule is a multi-specific antibody or a bi-specific antibody.

In one embodiment, the first and/or second labelled reagent comprises a target antigen of the multi-specific binding molecule.

In one embodiment, the first and/or second labelled reagent is a fluorescently-labelled reagent.

In one embodiment, the first and/or second labelled reagent is an anti-idiotypic antibody-fluorophore conjugate.

In one embodiment, the host cells are mammalian cells.

In one embodiment, the host cells are cultured in a volume of between about 0.3 nanoliters and about 500 microliters.

### Description of the Figures

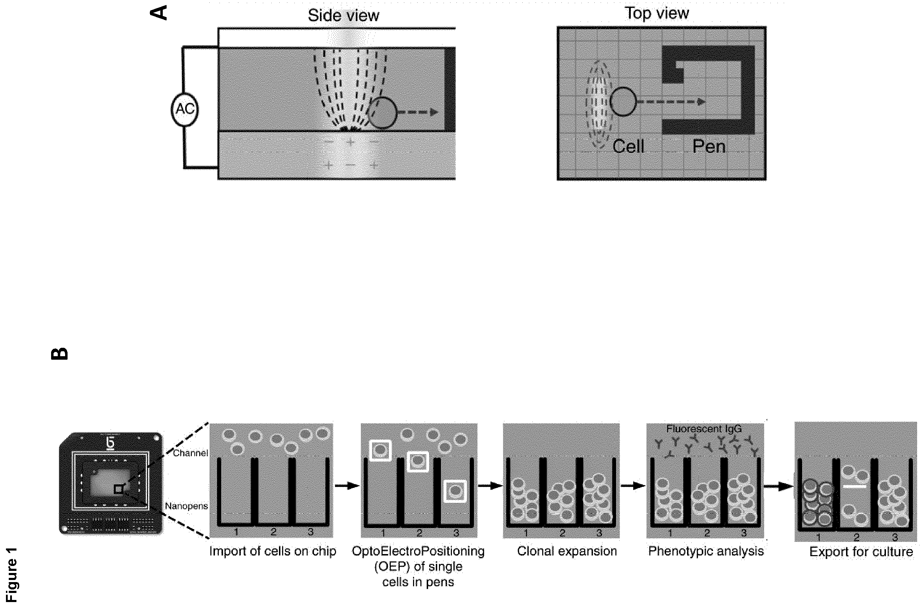

Figure 1: Diagram showing (A) an individual sequestration pen in a microfluidic device such as the Berkeley Lights Beacon as well as a representation of optoelectric positioning; and (B) a typical workflow using the Beacon to select cells. The antibodies shown are as an example, labelled anti-idiotypic antibodies that bind to a correctly formed binding site in the multi-specific antibodies of interest that are produced by the cell clones in each pen.

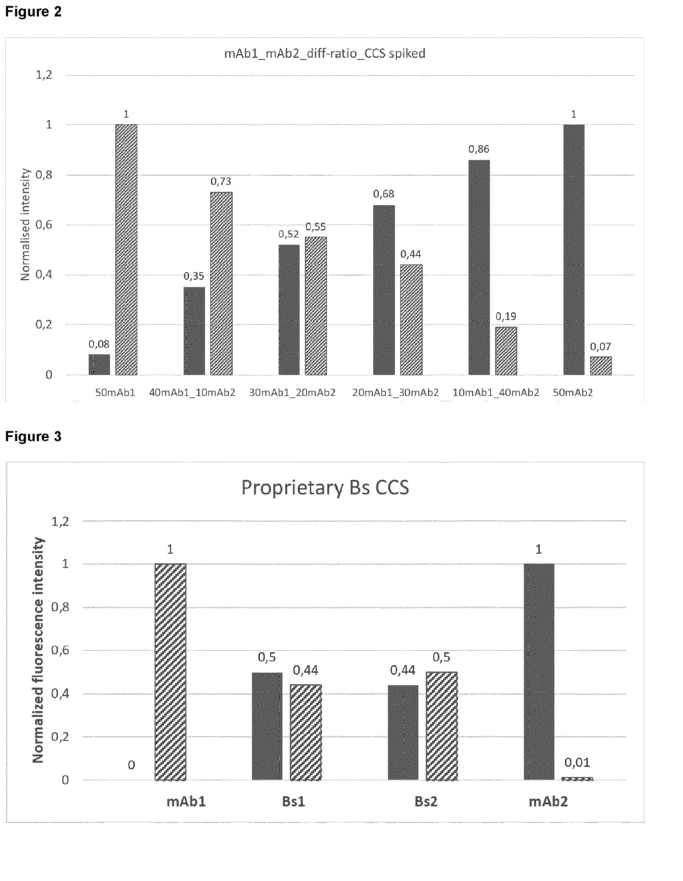

Figure 2: Normalized intensities of varying ratios of mAbs 'spiked' in null CCS (cell culture supernatant). Different ratios of monoclonal antibodies mAb1 and mAb2 were spiked into cell culture supernatant. mAb1 and mAb2 were immobilized in protein A-coated wells of a 96-well plate, washed and the ratio of binding sites determined by incubation with fluorescently labelled antigens specifically binding the binding sites of the monoclonal antibodies.
Figure 3: Normalized intensities of microplate plate assay to determine the heterogeneity of bispecific antibodies with different architectures. Cell culture supernatant from host cells producing bispecific antibodies of two different architectures (Bs1 and Bs2) was added to protein A-coated wells of a 96-well plate, thereby immobilizing the bispecific antibodies. Antibodies were washed and the ratio of binding sites determined by incubation with fluorescently labelled antigens specifically binding the individual binding sites of the bispecific antibodies.

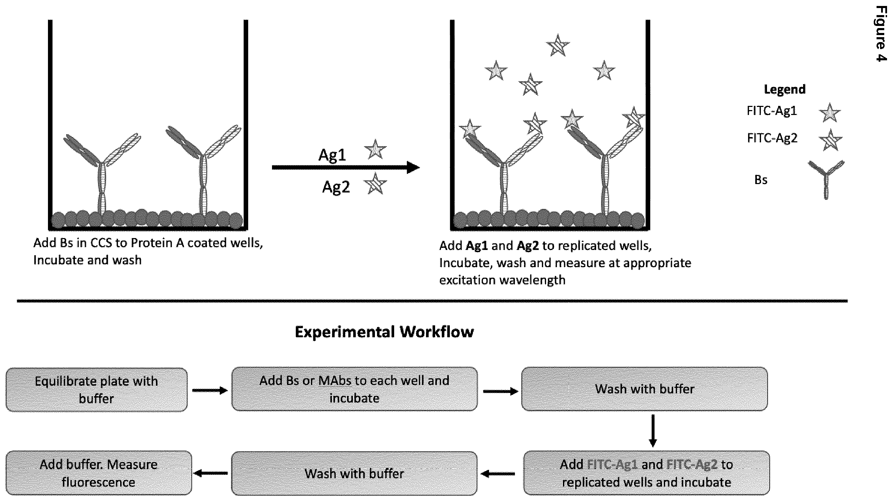

Figure 4: Schematic workflow of fluorescence-based microplate plate assay to determine the heterogeneity of bispecific antibodies (Ag1 = mAb1-binding Antigen 1; Ag2 = mAb2-binding Antigen 2; FITC = fluorescein isothiocyanate; Bs = bispecific antibody; CCS = cell culture supernatant). For each well, the fluorescence intensity is determined and normalized for each of the reagents (i.e., labelled antigens), to obtain an intensity score for each reagent and well. A pairing score may be calculated by dividing the lowest intensity score by the highest intensity score for each well. A well comprising only perfectly assembled bispecific antibodies will yield identical intensity scores for each reagent and therefore a pairing score of 1 (i.e., 100%). Mispairing will result in different intensity scores for each reagent and thus pairing scores below 1, e.g., 0.95, 0.9 or 0.8.

### Detailed Description of the Invention

### Methods of selecting a cell for expression of a multi-specific binding molecule

In a first aspect, the present invention provides a method of selecting a cell for expression of a multi-specific binding molecule comprising the following steps:
(a) providing a population of host cells comprising one or more nucleic acid sequences encoding at least two polypeptides,
   wherein the polypeptides are expressed by the host cells; and
   wherein a first polypeptide forms a first binding site for a target molecule, and a second polypeptide forms a second binding site for a target molecule, which is different to the first binding site;
(b) contacting the host cells with (i) a first labelled reagent that binds selectively to the first binding site, and (ii) a second labelled reagent that binds selectively to the second binding site, wherein the labels for the first and second reagents are different;
(c) measuring the level of first labelled reagent and second labelled reagent bound to their respective binding sites; and
(d) selecting one or more host cells expressing a multi-specific binding molecule based on a comparison of the two levels measured in step (c); and
wherein the host cells are cultured in a microfluidic device, wherein the microfluidic device comprises a microfluidic channel to which a plurality of sequestration pens are fluidically connected, wherein the host cells are loaded into the microfluidic device such that a plurality of the sequestration pens are each loaded with one host cell.

In some embodiments, the first polypeptide and a third polypeptide together form the first binding site for a target molecule, and the second polypeptide and a fourth polypeptide together form the second binding site for a target molecule, which is different to the first binding site.

The first, second, third and fourth polypeptides may be referred to herein as polypeptides (i), (ii), (iii) and (iv), respectively.

In some embodiments, when correctly assembled, the multi-specific binding molecule comprises one copy of each, the first and the second polypeptide, or one copy of each, the first, second, third and fourth polypeptide.

In some embodiments, the at least two polypeptides or the at least four polypeptides are heterologous polypeptides. As used herein, a heterologous polypeptide is a polypeptide that is not natively expressed by the host cells, i.e., a polypeptide that is derived from a different organism or cell type as compared to the host cells.

In some embodiments, the third and fourth polypeptides are the same.

In some embodiments, the target molecule to which the first binding site (formed by the first polypeptide or by the first and third polypeptides) binds, is different to the target molecule to which the second binding site (formed by the second polypeptide or by the second and fourth polypeptides) binds.

In some embodiments, the first binding site is a first immunoglobulin antigen binding region, and the second binding site is a second immunoglobulin antigen binding region.

In some embodiments, the at least two polypeptides each comprise three immunoglobulin complementarity determining regions (CDRs). For example, the first polypeptide may comprise a first set of three CDRs, and the second polypeptide may comprise a second set of three CDRs. The third polypeptide may comprise a third set of three CDRs, and the fourth polypeptide may comprise a fourth set of three CDRs.

In some embodiments, the first polypeptide comprises a first immunoglobulin heavy chain Fab region and the second polypeptide comprises a second immunoglobulin heavy chain Fab region. In further embodiments, the third polypeptide comprises a first immunoglobulin light chain Fab region and the fourth polypeptide comprises a second immunoglobulin light chain Fab region. In yet further embodiments, the first polypeptide comprises a first immunoglobulin heavy chain Fab region, the second polypeptide comprises a second immunoglobulin heavy chain Fab region, the third polypeptide comprises a first immunoglobulin light chain Fab region and the fourth polypeptide comprises a second immunoglobulin light chain Fab region.

In some embodiments, at least one of the polypeptides comprises a signal peptide that leads to secretion of the multi-specific binding molecule from the host cells.

In some embodiments, the multi-specific binding molecules are secreted by the host cells.

In some embodiments, the multi-specific binding molecule is a multi-specific antibody or a bi\-specific antibody.

The first and second labelled reagent generally comprise a moiety that binds selectively to the first or second binding site, respectively, and a label.

In some embodiments, the first and/or second labelled reagent comprises a target antigen of the multi-specific binding molecule, i.e., the labelled reagents comprise the target molecule to which the first and the second binding site bind, or a fragment thereof. Preferably, the first and second labelled reagents comprise the target antigens of the multi-specific binding molecule

In some embodiments, the first and/or second labelled reagent is an anti-idiotypic antibody or antibody fragment (e.g., Fab or scFv molecules). Preferably, the first and second labelled reagents are anti-idiotypic antibodies or antibody fragments.

In some embodiments, the first and/or second labelled reagent is a fluorescently-labelled reagent. Preferably, the first and second labelled reagents are fluorescently-labelled reagents.

In some embodiments, the first and/or second labelled reagent is an anti-idiotypic antibody-fluorophore conjugate. Preferably, the first and second labelled reagents are anti-idiotypic antibody-fluorophore conjugates.

In some embodiments, the host cells are mammalian cells.

In some embodiments, the host cells are cultured in a volume of between about 0.3 nanoliters (nL) and about 500 microliters (µL). Preferably, the cells are cultured in a volume of between about 0.4 nanoliters and about 250 microliters, more preferably between about 0.5 nanoliters and about 200 microliters.

In some embodiments, the host cells are cultured in a Beacon^{®} Optofluidic System from Berkeley Lights.

### Multi-Specific Binding Molecules

The methods of the invention can be used to screen populations of the cells that express multi-specific binding molecules to identify high performing clones. A multi-specific binding molecule in the context of the present invention is a complex of two or more different polypeptide components that comprises at least two different binding sites which bind to target molecules. In other words, the present invention is applicable to molecular complexes where there are multiple components which could assemble in different ways with one another, and it is desired to maximise the correct assembly of the molecule.

Each polypeptide component comprises a binding region for a target molecule of interest. The binding region of each component can pair with a binding region of another component to form a binding site for the target molecule. The overall molecular complex has at least two different binding sites, which may be for a different site on the same target molecule or, more commonly, two different target molecules. Examples of target molecules include cell surface molecules, such as receptors, spike proteins, extracellular proteins or any antigen protein.

In some embodiments, the polypeptides are single-domain antibodies.

In some embodiments, the multi-specific binding molecule is a multi-specific antibody or a bi-specific antibody. In some embodiments, the multi-specific binding molecule is an IgG-like bispecific antibody such as a DVD-IgG, IgG-scFv-scFv, scFv4, IgG-Fab, IgG-VH/VL or DVI-IgG.

In some embodiments, the target molecule to which the first binding site (formed by the first polypeptide or by the first and third polypeptides) binds, is different to the target molecule to which the second binding site (formed by the second polypeptide or by the second and fourth polypeptides) binds.

A typical example of the multi-specific binding molecule is a bispecific antibody which commonly comprises two different heavy chains and two different light chains such that, by contrast to a naturally-occurring IgG antibody, has two different antigen binding regions. In some implementations, a common light chain is used and so there are only three different chains, i.e., the third and fourth polypeptides may comprise an immunoglobulin light chain Fab region and may be identical.

Some formats of bispecific antibodies do not include full length heavy or light chains and therefore the polypeptide chains may comprise immunoglobulin antigen binding regions (i.e., the complementarity determining regions (CDRs)) optionally with some associated immunoglobulin constant region sequences. For example, the polypeptide chains may comprise the Fab regions of an immunoglobulin without any Fc regions - these may be omitted or substituted with alternative sequences that provide for pairing such as other types of polypeptides that dimerize (e.g., a leucine zipper). Naturally occurring immunoglobulins generally have 3 CDRs on each polypeptide chain such that 6 CDRs form an antigen binding site. Accordingly, the immunoglobulin antigen binding region of each of the polypeptides typically includes 3 CDRs.

In another embodiment, the polypeptide chains are complete, or substantially complete immunoglobulin chains, such as immunoglobulin light chains or heavy chains. The sequences may be engineered to enhance correct heavy chain to heavy chain pairing by substitutions in the Fc region e.g. mutations that create cysteine residues to provide for disulphide linkages; "knobs-in-holes" type mutations such as the WSAV approach; and/or substitutions that direct electrostatic interactions (electrostatic steering).

The sequences may be engineered to enhance correct heavy chain to light chain pairing by substitutions in the Fab region (constant or variable domains), e.g., mutations that create cysteine residues to provide for disulphide linkages; electrostatic steering; and/or domain swapping, such as the CrossMAbs approach.

Other formats exist that provide for more than two different binding sites, e.g., three or four binding sites. Such approaches are well known in the art.

Various formats also exist where additional sequences are added to the immunoglobulin sequences, e.g., appended IgG-like bispecific antibodies such as DVD-IgG, IgG-scFv-scFv, scFv4, IgG-Fab, IgG-VH/VL, DVI-IgG; and fusions such as dock and lock (see Bratt et al., 2017, BioProcess International 15(11): 36-42).

### Host Cells

Any suitable host cell type may be used in the methods of the invention which can be genetically manipulated to express and secrete multi-specific binding molecules. Preferred host cells are those that can be used to express the multi-specific binding molecules on a large scale, for commercial production of the multi-specific molecule.

In some embodiments, the host cell is a eukaryotic cell, for example mammalian, yeast or insect cell.

In one embodiment, the host cell is a mammalian cell. Example species from which host cell can be derived include human, mouse, rat, Chinese hamster, Syrian hamster, monkey, ape, dog, horse, ferret, and cat.

In a particular embodiment, the mammalian host cell is a Chinese hamster ovary (CHO) cell. In one embodiment, the host cell is a CHO-K1 cell, a CHOK1SV cell, a DG44 CHO cell, a DUXB11 CHO cell, a CHO-S, a CHO GS knock-out cell, a CHOK1SV FUT8 knock-out cell, a CHOZN, or a CHO-derived cell. The CHO GS knock-out cell (e.g., GSKO cell) is, for example, a CHO-K1SV GS knockout cell (Lonza Biologics, plc). The CHO FUT8 knockout cell is, for example, the Potelligent^{®} CHOK1SV FUT8 knock-out (Lonza Biologics, plc).

Other mammalian host cells include HeLa, MDCK, HEK293, HEK293T, HT1080, H9, HepG2, MCF7, Jurkat, NIH3T3, PC12, PER.C6, BHK (baby hamster kidney), VERO, SP2/0, NS0, YB2/0, Y0, EB66, C127 and COS (e.g., COS1 and COS7).

In other embodiments, the host cell is a cell other than a mammalian cell, such as avian, fish, insect, plant, fungus, or yeast cell.

In embodiments, the eukaryotic cell is a lower eukaryotic cell such as, e.g., a yeast cell (e.g., *Pichia* genus (e.g., *Pichia pastoris, Pichia methanolica, Pichia kluyveri,* and *Pichia angusta), Komagataella* genus, *Saccharomyces* genus (e.g. *Saccharomyces cerevisae, Saccharomyces kluyveri, Saccharomyces uvarum*)*,* or *Kluyveromyces* genus (e.g. *Kluyveromyces lactis, Kluyveromyces marxianus*)*.* In some embodiments, the eukaryotic cell is of the species *Pichia pastoris.* Examples for *Pichia pastoris* strains include but are not limited to X33, GS115, KM71, KM71H, and CBS7435.

In embodiments, the eukaryotic cell is an insect cell (e.g., Sf9, Mimic^{™} Sf9, Sf21, High FiveTM (BT1-TN-5B1-4), or BT1-Ea88 cells).

Suitable host cells are commercially available, for example, from culture collections such as the DSMZ (Deutsche Sammlung von Mikroorganismen and Zellkulturen GmbH, Braunschweig, Germany) or the American Type Culture Collection (ATCC).

### Population of Host Cells Transformed with Nucleic Acid Sequences Expressing Multi-specific Binding Molecules

Nucleic acid sequences encoding the different components of the multi-specific binding molecules can be introduced into populations of host cells using techniques well known in the art. The sequences encoding the constituent polypeptide chains, operably linked to regulatory control elements that drive expression of the polypeptides in the host cells are typically present in one or more nucleic acid vectors. One or more of the polypeptides will also typically include a signal sequence that directs secretion of the polypeptide from the host. The vectors typically include one or more selectable markers to enable selection of host cells that have taken up the nucleic acid vectors. Example of selectable markers include dhfr and amino acid auxotrophy-based markers such as glutamine synthetase (GS).

Following cell selection for the presence of the introduced nucleic acid sequences into the host cells, a population of cells may be generated wherein a plurality of the cells comprise one or more nucleic acid sequences encoding (i) a first polypeptide comprising a first immunoglobulin antigen binding region, such as an immunoglobulin CDR; (ii) a second polypeptide comprising a second immunoglobulin antigen binding region, such as an immunoglobulin CDR; (iii) a third polypeptide comprising a third immunoglobulin antigen binding region, such as an immunoglobulin CDR; and (iv) a fourth polypeptide comprising a fourth immunoglobulin antigen binding region, such as an immunoglobulin CDR, wherein the first and third immunoglobulin antigen binding regions (e.g., CDRs) together form a first antigen binding site and the second and fourth immunoglobulin antigen binding regions (e.g., CDRs) together form a second antigen binding site different to the first antigen binding site.

The purpose of the selection process is to identify and develop further particular clones that produce high levels of correctly paired molecular complexes of interest (e.g., multi-specific binding molecules). Various genetic factors may mean that in a population of clones that have been transformed with the same sequences, not all clones behave in the same manner. The selection process is not based on testing for the binding of different sequences where different cells have different variants of the polypeptide components; rather, the cells in the population/pool have all been transformed with the same set of sequences. The variation comes from the host cells themselves. Thus, the population of cells that is subject to testing and selection contains the same sequences encoding the first, second, and, where applicable, third and fourth polypeptides. Due to transformation efficiencies, it is possible that not every cell in the population contains all of the sequences but those cells would in any case not be of interest.

The CDRs may be part of an immunoglobulin Fab region and accordingly the plurality of the cells may comprise (i) a first polypeptide comprising a first immunoglobulin heavy chain Fab region; (ii) a second polypeptide comprising a second immunoglobulin heavy chain Fab region; (iii) a third polypeptide comprising a first immunoglobulin light chain Fab region; and (iv) a fourth polypeptide comprising a second immunoglobulin light chain Fab region. The first immunoglobulin heavy chain Fab region and the first immunoglobulin light chain Fab region together form a first immunoglobulin antigen binding region; and the second immunoglobulin heavy chain Fab region and the second immunoglobulin light chain Fab region together form a second immunoglobulin antigen binding region different to the first immunoglobulin antigen binding region.

As noted further above, some approaches include a common chain such that there are 3 different polypeptides rather than 4 or more. Accordingly, one of the polypeptides (i) to (iv) may be identical to one or the others. For example, the third and the fourth polypeptide may be identical and there are in reality three different polypeptides: a first, a second and a third polypeptide such that the first polypeptide pairs with the third polypeptide for the first antigen binding site and the second polypeptide also pairs with the third polypeptide for the second antigen binding site.

The polypeptides may also comprise Fc regions to form full length heavy and light chains.

As discussed above, different regions of the polypeptides may have been modified to promote heavy chain-heavy chain pairing and/or heavy light chain pairing.

### Screening of the Population of Host Cells in a Suitable Device

The host cells to be screened for expression of a correctly assembled multi-specific binding molecule according to the methods of the invention are cultured in a microfluidic device.

Selection of one or more cells expressing a multi-specific binding molecule based on a comparison of the levels of labelled reagents bound to their respective binding sites is generally facilitated by loading individual cells into compartments of a device used for culturing of the cells (sequestration pen of the microfluidic device), such that, after clonal expansion, each compartment comprises a population of cells resulting from clonal expansion of a single cell. According to the invention, the host cells are loaded into compartments (sequestration pens) of the microfluidic device used for culturing of the cells, such that a plurality of the sequestration pens are each loaded with one host cell.

Preferably, the host cells are clonally expanded to obtain a plurality of host cell populations, each of which is genetically homogenous.

The fluidic device comprises a substrate and the channel and pens are part of a fluidic structure which is disposed on a surface of the substrate. Cells suspended in a liquid medium can flow along the channel and pass the sequestration pens. The device is configured to enable individual cells to be loaded into a sequestration pen so that each sequestration pen contains only one cell, and also to enable the cells within a particular sequestration pen to be released into the channel and collected. The pens may be between about 0.3 nanoliters and about 500 microliters in volume, for example between about 0.3 and about 10 nL in volume for a microfluidic device.

The sequestration pens may comprise a fluidic isolation structure comprising an isolation region having a single opening and a connection region fluidically connecting said isolation region to the channel, the connection region comprising a proximal opening into the channels.

The substrate may be tilted at a small angle from the horizontal so that cells settle to the bottom of the sequestration pens and away from the narrow single opening into the channel.

An example of microfluidic device technology for achieving this is the use of OptoElectricPositioning (OEP), such as the Berkeley Lights Inc., Emeryville, CA Beacon system, e.g. as described in US9,857,333; Mocciaro et al., 2018, Light-activated cell identification and sorting (lacis) for selection of edited clones on a nanofluidic device. Commun Biol. 1:41; and Le et al., 2018, A novel mammalian cell line development platform utilizing nanofluidics and optoelectro positioning technology. Biotechnol Prog. 34:1438-46. OEP is based on a microfluidic device which includes a transparent electrode on a silicon substrate with a fluidic chamber sandwiched between the two. The substrate is fabricated with an array of photosensitive transistors. When focused light hits the transistors and a voltage is applied, a non-uniform electric field is generated. This imparts a negative dielectrophoresis (DEP) force that repels particles (including cells) using light-induced OEP (Figure 1a). In the absence of targeted light, no force is generated. When light is shined on the photoconductive material, DEP force is generated and cells trapped inside light "cages" can be moved across the chamber. In addition, sequestration pens (termed NanoPens^{™} in the Berkeley Lights device) are integrated into the chip to isolate cells from each other, enabling on-chip culture of well-separated colonies emanating from single cells. After characterization, selected clones can be exported off the microfluidic device for further processing. The export is the reverse of the import process, where desired cells are moved using OEP from single sequestration pens into the main channel and flushed, for example, into a target well of a 96-well plate positioned inside a CO₂- and temperature-controlled incubator.

The microfluidic device may, as per any manufacturer's instructions, be pre-wetted with a suitable wetting solution that creates an environment compatible with the host cells and allows for good penning efficiency. The device can then be primed with cell culture media suitable for the growth of the host cells and protein expression.

Once cells have been loaded into the device and positioned into individual compartments (sequestration pens), the cells are incubated to allow for cell growth and clonal expansion, as well as the production of the multi-specific binding molecules. Monitoring of the different compartments can be used to ensure monoclonality in each compartment and also to ensure that the cells do not overfill the compartments prior to analysis of the multi-specific binding molecules.

Cells are then analysed by introduction into the device of reagents that bind specifically to a correctly formed first binding site or to a correctly formed second binding site in the multi-specific molecule. In one embodiment, the reagents comprise the corresponding target antigen for the binding sites, e.g., the target molecule or a fragment thereof. In another embodiment, the reagents are anti-idiotypic antibodies, including fragments thereof such as Fab, scFv molecules, specific to one of the correctly formed binding sites in the multi-specific molecule. Other reagents include aptamers - oligonucleotide or peptide molecules that have the requisite binding specificity.

The binding molecules each need to be specific for the different binding sites so that binding can be distinguished.

The reagents are typically labelled with a detectable label to enable binding of the reagent to its target to be measured in situ in the device. In one embodiment, the label is a chemiluminescent label. In another embodiment the label is a fluorescent label, such as a fluorophore or a fluorescent protein. In one embodiment, the detectable label is fluorescein or a derivative thereof (e.g., fluorescein isothiocyanate). Exemplary fluorescent proteins are blue fluorescent proteins such as BFP and mTagBFP, cyan fluorescent proteins such as ECFP and TagCFP, green fluorescent proteins such as EGFP and ZsGreen, yellow fluorescent proteins such as EYFP and ZsYellow, red fluorescent proteins such as mRFP and mCherry, far-red proteins such as E2-Crimson.

Each different reagent is labelled with a different detectable label.

Each reagent can be introduced into the device separately or at the same time, (which may depend on whether different labels are used as well as the ability of the imaging system to distinguish between different labels, such as different fluorescent (or chemiluminescent) signals, to enable simultaneous measurement).

In one embodiment, the first reagent is introduced into the device such that it is able to enter the compartments (sequestration pens) and contact the multi-specific molecule produced by the host cells in the compartment. The reagent is present in the compartments for a period of time to provide sufficient binding to the multi-specific molecule (e.g., 45 to 60 minutes). After a suitable washing step, if required, the binding of the first reagent to the first binding site in the molecule is determined, e.g., by fluorescent imaging of the device. A wash step is then used to remove the first reagent and the process is repeated with the second reagent and so on.

The extent of binding of the first and second reagents is determined e.g. using image analysis algorithms or scripts. This can be used to determine an intensity score (e.g., a normalized signal intensity) for each compartment (e.g., well or sequestration pen) for each reagent, adjusted as necessary to actual binding amounts depending on the performance of the labels used so that an accurate comparison of the intensity score for the different reagents can be made.

The intensity scores for each reagent are then compared to obtain a pairing score, e.g., a percentage obtained by dividing the lowest intensity score by the highest intensity score for each pen. A similar score, e.g., greater than or equal to 90 or 95%, indicates high levels of correctly paired chains since similar amounts of correctly formed first and second binding sites in the multi-specific molecule are present in the compartment (e.g., well or sequestration pen). Expressed in another way, if the intensities are within +/- 20%, such as +/- 10% or +/-5% of each other, then this would be considered a similar score. Conversely, a score outside of a similar score is indicative of significant levels of mispaired molecules, e.g., greater than +/- 20% referring to the calculations above. Accordingly, in one embodiment, levels of first labelled reagent and second labelled reagent bound to their respective binding sites that are within +/- 20% of each other (i.e., have intensity scores within +/- 20% of each other), preferably within +/- 10% of each other and most preferably within +/- 5% of each other indicate correctly-paired multi-specific binding molecules (i.e., multi-specific binding molecules comprising both a first binding site for a target molecule, and a second binding site for a target molecule).

Typically, cells in particular compartments are scored for productivity (total levels of multi-specific molecule production) since it is advantageous for industrial production for the cells to be able to produce high titers of the product of interest. The scoring may be a relative score between the various clones. The Beacon system provides SpotLight^{™} Human Fc and kappa light chain assays. These are fluorophores that bind to the Fc and/or the kappa light chain constant region and give a fluorescence signal directly proportional to the amount of antibody expressed in that particular clone. A "score" value is created by measuring the change in fluorescence in the or close to the neck of the pen in the (what they call diffusion gradient assay). A higher slope value means higher titre. The maximum score is normalized to 100 and the minimum to 0. The values in between are the percent of the total and the score is calculated from these values. The rQp is the relative production per cell of the last DiGr assay, i.e. the score divided by the number of cells.

Individual clones that meet the threshold for correct pairing, and typically the threshold for productivity can then be exported from the device, e.g., into 96-well plates, for further growth. Typically, selected clones are then further assessed to identify the highest producing clones (high Qp).

Clones may optionally be subject to further analysis to confirm the high levels of correct pairing. For example, assessment of purified molecule by limited digestion (e.g. with Lys-C) and mass spectroscopy (LC-MS) can be used to check for quantitate in more detail the levels and configuration of non-correctly paired variants.

### Applications for Selected Clones

Selected clones can then be used to establish stable cell lines for use in manufacturing recombinant multi-specific binding molecules of interest, for example at a scale of greater than 500 g per batch, e.g., in a bioreactor having a volume of at least 10 L such as at least about 100, 200, 500, 1000 or 2000 L.

The present invention will be illustrated further with reference to the following examples, which are non-limiting.

### EXAMPLES

### Example 1: Determination of heterogeneity of mAbs using a microfluidic Beacon system

### Materials

- Stable pool of cells expressing Antibody 1/ Antibody 2 bispecific antibody
- Anti Antibody 1 idiotypic antibody fluorophoreA-conjugated
- Antigen 1 fluorophoreA-conjugated
- Anti Antibody 2 idiotypic antibody fluorophoreB-conjugated
- Antigen 2 fluorophoreB-conjugated

### Methods

1. Prepare chips (wetting the Beacon^{™} OptoSelect^{™} chip with the Wetting Solution supplied by the manufacturer creates a cell-friendly environment that ensures good penning efficiency and enables cell growth) - Day 1
2. Assay calibration: Diffusion Gradient (DiGr) assay (protein yield), fluorophore A and fluorophore B reference images are acquired for each OptoSelect chip - Day 1
3. Prime chips and lines with cell culture media - Day 2
4. Loading cells: from cell load plate into specific Optoselect chip - Day 2
5. Culture growth - Day 2 to Day 5 morning
6. Assays: - Day 5 to Day 7
   a. Productivity assay (DiGr) followed by the minimum amount of time it takes the fluorophore to diffuse and disappear from the chip, then:
   b. Fluorophore A assay (fluorophore conjugated to anti idiotypic antibody A / fluorophore conjugated to antigen A), followed by the minimum amount of time it takes the fluorophore to diffuse and disappear from the chip, then:
   c. Fluorophore B assay (fluorophore conjugated to anti idiotypic antibody B / fluorophore conjugated to antigen B).
   Fluorescence imaging of the chips is performed by the Beacon system, which can capture bright field and fluorescence images across multiple time points. Analysis of the image using a script results in assigning an "intensity score" to each NanoPen^{™} based on the gradient of intensity in a specific area of the NanoPen: the relative yield is measured this way. A script then compares the intensity scores for each NanoPen for fluorophore A and fluorophore B, and generates a "pairing score" based on similarity of the levels of bound fluorophore, the higher the score the more similar the levels of bound fluorophores A and B, and the higher percentage of correctly paired heterodimeric antibody that particular clone is producing. Software to analyse the fluorescence images also includes free software such as ImageJ (available from the National Institutes of Health website), that measure intensity.
7. Export clones based on %heterodimerization and productivity. This can be based either on scoring information generated and stored in the Beacon system and used as a default for clone export (e.g. the top scoring clones for export will be the ones with higher %heterodimerization + higher productivity) or top clone identification information uploaded into the Beacon system
8. Clone growth and progression in 96 well plates.

This procedure can also be performed with a single fluorophore since measurements can be taken sequentially and the first reagent will then be washed out before the second reagent is introduced.

### Example 2: Determination of heterogeneity of varying ratios of mAbs 'spiked' in null CCS (cell culture supernatant)

### Materials

Protein A coated 96-well plates
DPBS+0.05%P20 (Dulbecco's phosphate buffered saline, comprising 0.05% (v/v) polysorbate 20 as surfactant)
mAb1
mAb2
mAb1-binding Antigen 1 FITC-conjugated (FITC = fluorescein isothiocyanate)
mAb2-binding Antigen 2 FITC-conjugated

### Method

1- Equilibrate Protein A coated 96-well plate with 200 µL of DPBS+0.05%P20 per well for 10 min.
2- Remove buffer and add different ratios of mAb1 and mAb2 spiked in null CCS.
3- Incubate at RT for 30-35 min and remove the supernatant.
4- Wash wells carrying samples with 200 µL DPBS+0.05%P20 per well, three times.
5- Add 100 µL of DPBS+0.05%P20 to each well.
6- Add 1.4 µL of mAb1-binding Antigen 1 FITC-conjugated and mAb2-binding Antigen 2 FITC-conjugated to the respective wells and incubate for a further 30min.
7- Wash wells with 200 µL DPBS+0.05%P20 each, three times.
8- Add 100 µL of DPBS+0.05%P20 to each well and measure fluorescence intensity using a SpectraMax M5e plate reader (For measuring fluorescence, an Ex/Em = 488/535 nm was used. 50 flashes per read was chosen in the settings. All measurements were made at 25°C. Data were exported and analysed in Microsoft Excel).

### Data Analysis

Mean and standard deviation (SD) of triplicate measurements were obtained. Fluorescence intensity obtained from null-CCS were subtracted from those obtained from the rest of the samples. Intensities obtained for mAb1-binding Antigen 1 FITC-conjugated were normalized against mAb1, and those obtained for mAb1-binding Antigen 2 FITC-conjugated were normalized against mAb2 (Tables 1 and 2). Normalized intensities were plotted as bar graphs (Fig. 2).

**Table 1**

| blank | 50 nM mAb1 | 40 nM mAb1 - 10 nM mAb2 | 30 nM mAb1 - 20 nM mAb2 | 20 nM mAb1 - 30 nM mAb2 | 10 nM mAb1 - 40 nM mAb2 | 50 nM mAb2 |
|---|---|---|---|---|---|---|
| 0,575 | 0,582 | 3,227 | 3,649 | 5,313 | 6,768 | 7,696 |
| 0,55 | 0,582 | 2,441 | 4,076 | 4,6 | 6,111 | 7,32 |
| 0,587 | 0,558 | 2,156 | 3,834 | 5,011 | 6,114 | 7,091 |
| 0,57 | 0,57 | 2,61 | 3,85 | 4,97 | 6,33 | 7,37 |
| 0,02 | 0,01 | 0,45 | 0,17 | 0,29 | 0,31 | 0,25 |
| 0,00 | 0,00 | 2,04 | 3,28 | 4,40 | 5,76 | 6,80 |
| Normalized to mAb2 | 0,08 | 0,35 | 0,52 | 0,68 | 0,86 | 1,00 |

**Table 2**

| blank | 50 nM mAb1 | 40 nM mAb1 - 10 nM mAb2 | 30 nM mAb1 - 20 nM mAb2 | 20 nM mAb1 - 30 nM mAb2 | 10 nMmAb1 - 40 nM mAb2 | 50 nM mAb2 |
|---|---|---|---|---|---|---|
| 0,547 | 7,855 | 5,511 | 4,12 | 3,794 | 1,415 | 0,571 |
| 0,6 | 7,598 | 5,228 | 4,313 | 3 | 1,337 | 0,52 |
| 0,554 | 6,984 | 5,707 | 3,863 | 3,178 | 1,431 | 0,521 |
| 0,57 | 7,48 | 5,48 | 4,10 | 3,32 | 1,39 | 0,54 |
| 0,02 | 0,37 | 0,20 | 0,18 | 0,34 | 0,04 | 0,02 |
| 0,00 | 6,91 | 4,92 | 3,53 | 2,76 | 0,83 | -0,03 |
| Normalized to mAb1 | 1,00 | 0,73 | 0,55 | 0,44 | 0,19 | 0,07 |

Data show that the plate assay can be used to assess the degree of heterogeneity in various CCS samples.

### Example 3. Fluorescence-based 96-well plate assay to determine the heterogeneity of bispecific antibodies with different architectures.

### Materials

Protein A coated 96-well plates
DPBS+0.05%P20
Supernatant of cell culture expressing proprietary bispecific antibody bs1 (composed of mAb1 and mAb2, bispecific architecture a)
Supernatant of cell culture expressing proprietary bispecific antibody bs2 (composed of mAb1 and mAb2, bispecific architecture b)
mAb1-binding Antigen 1 FITC-conjugated
mAb2-binding Antigen 2 FITC-conjugated

### Method

1- Equilibrate Protein A coated 96-well plate with 200 µL of DPBS+0.05%P20 per well for 10min.
2- Remove buffer and add 50 nM of samples in CCS to the appropriate wells. The concentration of the samples was adjusted by diluting the stocks (Day 4) with null CCS.
3- Incubate at RT for 30-35 min and remove the supernatant
4- Wash wells carrying samples with 200 µL DPBS+0.05%P20 per well, three times.
5- Add 100 µL of DPBS+0.05%P20 to each well.
6- Add 1.4 µL of mAb1-binding Antigen 1 FITC-conjugated and mAb2-binding Antigen 2 FITC-conjugated to the respective wells and incubate for a further 30 min
7- Wash wells with 200 µL DPBS+0.05%P20 each, three times.
8- Add 100 µL of DPBS+0.05%P20 to each well and measure fluorescence intensity using the SpectraMax M5e plate reader (For measuring fluorescence, an Ex/Em = 488/535 nm was used. 50 flashes per read was chosen in the settings. All measurements were made at 25°C. Data were exported and analysed in MS Excel).

### Data Analysis

Mean and SD of triplicate measurements were obtained. Fluorescence intensity obtained from null-CCS were subtracted from those obtained from the rest of the samples. Intensities obtained for mAb1-binding Antigen 1 FITC-conjugate (Ag1-FITC) were normalized against mAb1, and those obtained for mAb1-binding Antigen 2 FITC-conjugate (Ag2-FITC) were normalized against mAb2 (Table 3). Normalized intensities were plotted as bar graphs (Fig. 3).

The plate-based format to distinguish the degree of heterogeneity in bispecific cultures is feasible. When proprietary bs molecules were tested using this method the ratio of intensities obtained for mAb1-binding Antigen 1 FITC-conjugated and mAb2-binding Antigen 2 FITC-conjugated correlates with LCMS data obtained by those specific molecules.

The workflow of the method is schematically depicted in Fig. 4.

**Table 3**

| | **0.05 µM mAb2** | **0.05 µM Bs1** | **0.05 µM Bs2** | **0.05 µM mAb1** | **CCS** |
|---|---|---|---|---|---|
| Ag1-FITC | 0,556 | 3,316 | 2,977 | 5,402 | 0,545 |
| Ag1-FITC | 0,55 | 2,576 | 2,423 | 5,196 | 0,559 |
| Ag1-FITC | 0,539 | 2,818 | 2,399 | 5,068 | 0,558 |
| Mean | 0,55 | 2,90 | 2,60 | 5,22 | 0,55 |
| Std dev | 0,01 | 0,38 | 0,33 | 0,17 | 0,01 |
| Corrected mean | -0,01 | 2,35 | 2,05 | 4,67 | 0,00 |
| normalized to mAB2 | 0,00 | 0,50 | 0,44 | 1,00 | |
| Ag2-FITC | 4,923 | 2,332 | 2,408 | 0,573 | 0,569 |
| Ag2-FITC | 4,732 | 2,489 | 2,701 | 0,588 | 0,547 |
| Ag2-FITC | 4,394 | 2,347 | 2,796 | 0,693 | 0,554 |
| Mean | 4,68 | 2,39 | 2,64 | 0,62 | 0,56 |
| Std dev | 0,27 | 0,09 | 0,20 | 0,07 | 0,01 |
| Corrected mean | 4,13 | 1,83 | 2,08 | 0,06 | 0,00 |
| Normalized to mAB1 | 1 | 0,44 | 0,50 | 0,01 | |

## Claims

1. A method of selecting a cell for expression of a multi-specific binding molecule comprising the following steps:
(a) providing a population of host cells comprising one or more nucleic acid sequences encoding at least two polypeptides,
wherein the polypeptides are expressed by the host cells; and
wherein a first polypeptide forms a first binding site for a target molecule, and a second polypeptide forms a second binding site for a target molecule, which is different to the first binding site;
(b) contacting the host cells with (i) a first labelled reagent that binds selectively to the first binding site, and (ii) a second labelled reagent that binds selectively to the second binding site, wherein the labels for the first and second reagents are different;
(c) measuring the level of first labelled reagent and second labelled reagent bound to their respective binding sites; and
(d) selecting one or more host cells expressing a multi-specific binding molecule based on a comparison of the two levels measured in step (c); and
wherein the host cells are cultured in a microfluidic device, wherein the microfluidic device comprises a microfluidic channel to which a plurality of sequestration pens are fluidically connected, wherein the host cells are loaded into the microfluidic device such that a plurality of the sequestration pens are each loaded with one host cell.

2. The method of claim 1,
wherein the first polypeptide and a third polypeptide together form the first binding site for a target molecule, and the second polypeptide and a fourth polypeptide together form the second binding site for a target molecule, which is different to the first binding site.

3. The method of claim 2, wherein the third and fourth polypeptides are the same.

4. The method of any one of the preceding claims, wherein the multi-specific binding molecules are secreted by the host cells.

5. The method of any one of the preceding claims, wherein the multi-specific binding molecule is a multi-specific antibody or a bi-specific antibody.

6. The method of any one of the preceding claims, wherein the first and/or second labelled reagent comprises a target antigen of the multi-specific binding molecule.

7. The method of any one of the preceding claims, wherein the first and/or second labelled reagent is a fluorescently-labelled reagent.

8. The method of any one of the preceding claims, wherein the first and/or second labelled reagent is an anti-idiotypic antibody-fluorophore conjugate.

9. The method of any one of the preceding claims, wherein the host cells are mammalian cells.

10. The method of any one of the preceding claims, wherein the host cells are cultured in a volume of between 0.3 nanoliters and 500 microliters.

## Patentansprüche

1. Verfahren zum Auswählen einer Zelle zur Expression eines multispezifischen Bindungsmoleküls, umfassend die folgenden Schritte:
(a) Bereitstellen einer Population von Wirtszellen, die eine oder mehrere Nukleinsäuresequenzen umfassen, die mindestens zwei Polypeptide kodieren,
wobei die Polypeptide von den Wirtszellen exprimiert werden; und
wobei ein erstes Polypeptid eine erste Bindungsstelle für ein Zielmolekül bildet und ein zweites Polypeptid eine zweite Bindungsstelle für ein Zielmolekül bildet, die sich von der ersten Bindungsstelle unterscheidet;
(b) Inkontaktbringen der Wirtszellen mit (i) einem ersten markierten Reagenz, das selektiv an die erste Bindungsstelle bindet, und (ii) einem zweiten markierten Reagenz, das selektiv an die zweite Bindungsstelle bindet, wobei die Markierungen für das erste und das zweite Reagenz unterschiedlich sind;
(c) Messen des Niveaus des ersten markierten Reagenz und zweiten markierten Reagenz, die an ihre jeweiligen Bindungsstellen gebunden sind; und
(d) Auswählen einer oder mehrerer Wirtszellen, die ein multispezifisches Bindungsmolekül exprimieren, basierend auf einem Vergleich der zwei in Schritt (c) gemessenen Niveaus; und
wobei die Wirtszellen in einer mikrofluidischen Vorrichtung kultiviert werden, wobei die mikrofluidische Vorrichtung einen mikrofluidischen Kanal umfasst, mit dem eine Vielzahl von Sequestrierungspens fluidisch verbunden sind, wobei die Wirtszellen derart in die mikrofluidische Vorrichtung geladen werden, dass eine Vielzahl der Sequestrierungspens jeweils mit einer Wirtszelle beladen werden.

2. Verfahren nach Anspruch 1,
wobei das erste Polypeptid und ein drittes Polypeptid zusammen die erste Bindungsstelle für ein Zielmolekül bilden und das zweite Polypeptid und ein viertes Polypeptid zusammen die zweite Bindungsstelle, die sich von der ersten Bindungsstelle unterscheidet, für ein Zielmolekül bilden.

3. Verfahren nach Anspruch 2, wobei das dritte und das vierte Polypeptid gleich sind.

4. Verfahren nach einem der vorstehenden Ansprüche, wobei die multispezifischen Bindungsmoleküle von den Wirtszellen abgesondert werden.

5. Verfahren nach einem der vorstehenden Ansprüche, wobei das multispezifische Bindungsmolekül ein multispezifischer Antikörper oder ein bispezifischer Antikörper ist.

6. Verfahren nach einem der vorstehenden Ansprüche, wobei das erste und/oder zweite markierte Reagenz ein Zielantigen des multispezifischen Bindungsmoleküls umfasst.

7. Verfahren nach einem der vorstehenden Ansprüche, wobei das erste und/oder zweite markierte Reagenz ein fluoreszenzmarkiertes Reagenz ist.

8. Verfahren nach einem der vorstehenden Ansprüche, wobei das erste und/oder zweite markierte Reagenz ein antiidiotypisches Antikörper-Fluorophor-Konjugat ist.

9. Verfahren nach einem der vorstehenden Ansprüche, wobei die Wirtszellen Säugetierzellen sind.

10. Verfahren nach einem der vorstehenden Ansprüche, wobei die Wirtszellen in einem Volumen zwischen 0,3 Nanolitern und 500 Mikrolitern kultiviert werden.

## Revendications

1. Procédé de sélection d'une cellule pour l'expression d'une molécule de liaison multispécifique comprenant les étapes suivantes :
(a) la fourniture d'une population de cellules hôtes comprenant une ou plusieurs séquences d'acide nucléique codant au moins deux polypeptides,
dans lequel les polypeptides sont exprimés par les cellules hôtes ; et
dans lequel un premier polypeptide forme un premier site de liaison pour une molécule cible, et un deuxième polypeptide forme un second site de liaison pour une molécule cible, qui est différent du premier site de liaison ;
(b) la mise en contact des cellules hôtes avec (i) un premier réactif marqué qui se lie sélectivement au premier site de liaison, et (ii) un second réactif marqué qui se lie sélectivement au second site de liaison, dans lequel les marqueurs des premier et second réactifs sont différents ;
(c) la mesure du niveau du premier réactif marqué et du second réactif marqué liés à leurs sites de liaison respectifs ; et
(d) la sélection d'une ou plusieurs cellules hôtes exprimant une molécule de liaison multispécifique sur la base d'une comparaison des deux niveaux mesurés à l'étape (c) ; et
dans lequel les cellules hôtes sont cultivées dans un dispositif microfluidique, dans lequel le dispositif microfluidique comprend un canal microfluidique auquel sont raccordés fluidiquement une pluralité d'enclos de séquestration, dans lequel les cellules hôtes sont chargées dans le dispositif microfluidique de sorte qu'une pluralité des enclos de séquestration soient chacun chargés d'une cellule hôte.

2. Procédé selon la revendication 1,
dans lequel le premier polypeptide et un troisième polypeptide forment ensemble le premier site de liaison pour une molécule cible, et le deuxième polypeptide et un quatrième polypeptide forment ensemble le second site de liaison pour une molécule cible, qui est différent du premier site de liaison.

3. Procédé selon la revendication 2, dans lequel les troisième et quatrième polypeptides sont identiques.

4. Procédé selon l'une quelconque des revendications précédentes, dans lequel les molécules de liaison multispécifiques sont sécrétées par les cellules hôtes.

5. Procédé selon l'une quelconque des revendications précédentes, dans lequel la molécule de liaison multispécifique est un anticorps multispécifique ou un anticorps bispécifique.

6. Procédé selon l'une quelconque des revendications précédentes, dans lequel le premier et/ou le second réactif marqué comprend un antigène cible de la molécule de liaison multispécifique.

7. Procédé selon l'une quelconque des revendications précédentes, dans lequel le premier et/ou le second réactif marqué est un réactif marqué par fluorescence.

8. Procédé selon l'une quelconque des revendications précédentes, dans lequel le premier et/ou le second réactif marqué est un conjugué anticorps anti-idiotypique-fluorophore.

9. Procédé selon l'une quelconque des revendications précédentes, dans lequel les cellules hôtes sont des cellules de mammifères.

10. Procédé selon l'une quelconque des revendications précédentes, dans lequel les cellules hôtes sont cultivées dans un volume compris entre 0,3 nanolitres et 500 microlitres.
